# EUROPEAN PATENT APPLICATION

(11) **EP 3 064 131 A1**
(43) Date of publication of application: **07.09.2016**
(21) Application number: 16154006.7
(22) Date of filing: 03.02.2016
(51) Int. Cl.: A61B 5/08, A61N 1/36

(54) **COMBINED VAGUS-PHRENIC NERVE STIMULATION APPARATUS**

(30) Priority: 03.03.2015 US 201562127296 P
(71) Applicant: BIOTRONIK SE & Co. KG, 12359 Berlin (DE)
(72) Inventor: Baru, Marcelo, Tualatin, OR Oregon 97062 (US)
(74) Representative: Galander, Marcus

(57) **Abstract**

The invention refers to by an implantable pulse generator (IPG) that is connected or can be connected to a stimulation lead having at least one stimulation electrode. This implantable pulse generator comprises a respiration activity sensing unit that is configured to generate a signal that represents a respiration activity and
at least one stimulation unit that is configured to generate electric stimulation pulses for nerve stimulation. The implantable pulse generator further comprises a control unit that is configured to trigger delivering of electric stimulation pulses via said at least one stimulation electrode, wherein triggering of the stimulation pulses depends on the signal that represents a respiration activity.

## Description

The invention relates to an implantable stimulation system comprising an implantable pulse generator (IPG) and a stimulation lead that is connected to the IPG and that has at least one stimulation electrode for delivery of stimulation pulses. The invention further relates to an IPG for such system and a stimulation lead for such system having at least one stimulation electrode. A further aspect of the invention relates to a method for vagus nerve stimulation.

In particular, the disclosed invention describes an implantable apparatus for combined Vagus-Phrenic Nerve Stimulation (VNS-PhrNS) therapy particularly suitable for the treatment of patients suffering from Congestive Heart Failure (CHF) with Central Sleep Apnea (CSA) syndrome.

Transvascular stimulation of a vagus nerve via a catheter, for the purpose of heart rate reduction (parasympathetic drive), was first reported by Thompson et al. in 1998 [Thompson et al. "Bradycardia induced by intravascular versus direct stimulation of the vagus nerve", Annals of Thoracic Surgery, 65(3), 637-42, 1998]. A few years later, Hasdemir et al. [Hasdemir et al. "Endovascular stimulation of autonomic neural elements in the superior vena cava using a flexible loop catheter", Japanese Heart Journal, 44(3), 417-27, 2003] investigated the use of a flexible loop with multiple contacts in the superior vena cava (SVC). Invariably, this work showed stimulation at anterior sites resulted in phrenic nerve stimulation whereas posterior site stimulation was associated with effect in sinus cycle length and atrioventricular conduction while avoiding phrenic nerve stimulation.

Transvascular stimulation of the phrenic nerves, on the other hand, dates back to the 1950s [Doris J. W. Escher et al. "Clinical control of respiration by transvenous phrenic pacing",

Trans. Amer. Soc. Artif. Int. Organs, Vol. XIV, 192-197, 1968]. WO2008/092246 A1 discloses a stimulation device with a single endovascular lead having multiple electrodes which allows stimulation of a vagus nerve and/or a phrenic nerve. Phrenic nerve stimulation to regulate breathing and positioning fine-tuning of the electrode array in the internal jugular vein (IJV) by observing the patient's breathing are disclosed.

US 8,433,412 B1 discloses a lead-electrode system for use with an Implantable Medical Device (IMD) configured to monitor and/or treat both cardiac and respiratory conditions. More particularly, embodiments of the invention relate to a lead-electrode configuration of a combination IMD that combines therapies such as cardiac pacing, respiratory sensing, phrenic nerve stimulation, defibrillation, and/or biventricular pacing, referred as Cardiac Resynchronization Therapy (CRT). In accordance with embodiments of such prior art, stimulation and/or sensing leads may be placed in a small pericardiophrenic vein, a brachiocephalic vein, an azygos vein, a thoracic intercostal vein, or other thoracic vein that affords proximity to the phrenic nerve for stimulation. Respiration sensing may be obtained via transthoracic impedance.

US 8,630,704 B2 discloses utilizing measurement of respiratory stability of instability during sleep or rest as a feedback to control stimulation of an autonomic neural target (e.g. vagus nerve stimulation).

Almost half of the CHF patient population suffers from CSA. Although CRT has become the standard device therapy for the treatment of NYHA class III or IV heart failure patients with left ventricular ejection fractions (LVEF) ≤ 35 % and QRS ≥ 130 ms, only 7% of all eligible CHF patients receive the device [see http://www.ncbi.nlm.nih.gov/pmc/articles/PMC3493802/] and approximately 30% of those who receive it are classified as non-responders [see http://circ.ahaiournals.org/content/117 /20/2608.abstract]. Hence, a combination device as proposed by US 8,433,412 B1 is not the most appropriate for the treatment of CHF with CSA.

Vagus nerve stimulation recently emerged as a potential progression-preventing and treatment option for CHF patients. Experimental data have demonstrated that stimulation of a vagus nerve at the cervical level is able to reverse ventricular remodeling of the failing heart. There is also evidence that increasing parasympathetic activity may stimulate the production of nitric oxide, and reduce the devastating inflammatory process involved in heart failure. Present vagus nerve stimulation apparatuses for CHF involve an implanted nerve cuff electrode that connects via wires to an IPG in the patient's chest. A standard pacemaker sensing lead in the ventricle has been proposed in prior art for the purpose of synchronous delivery of vagus nerve stimulation pulses in the cardiac refractory period although other prior art apparatuses operate asynchronously to the cardiac cycle. Stimulation of both the right and left vagus nerves are disclosed in prior art for CHF treatment.

At the cervical level, the vagus nerve bundle includes large-diameter fibers that innervate the muscles of the larynx and pharynx in addition to the target parasympathetic cardiac fibers. Since the former present lower threshold to stimulation compared to the latter, vagus nerve stimulation therapy delivery for CHF requires the utilization of special waveforms and electrode arrangements that preferentially target the cardiac fibers. Although side effects (e.g. coughing) are reduced with these special stimulation techniques, they are not fully eliminated which is a drawback of this stimulation approach.

Transvascular stimulation of the vagus nerve cardiac branches is very appealing as the implantation of endovascular leads is well known by Physicians dealing with CHF patients. Chronically implanting a lead in a large major vein as proposed by WO2008/092246 A1 requires a suitable anchoring solution not described in such prior art. Unfortunately, the absence of data supporting the safety and efficacy of SVC filters for upper-extremity Deep Vein Thrombosis (DVT) [see http://www.jvir.org/article/51051-0443%2810%2900208-3/abstract] have precluded the chronic implantation of endovascular leads in large veins for the purpose of nerve stimulation. There is no suitable solution in prior art that satisfactorily addresses this issue.

US 8,433,412 B1 does not disclose stimulation of the vagus nerve cardiac branches, although possible via a lead in the left superior intercostal or azygos vein. In such prior art, an "implantable respiration lead" is proposed for transvascularly stimulating a phrenic nerve. Embodiments of the respiration lead may be installed in a pericardiophrenic vein, a brachiocephalic vein, an IJV, a superior intercostal vein, the SVC, or other appropriate locations. Claims however only reference the azygos vein.

It is an object of the invention to provide a nerve stimulation apparatus that advances the state-of-the-art in the management/treatment of Congestive Heart Failure (CHF) patients in particular those who also suffer from Central Sleep Apnea (CSA) syndrome.

It is a further object of the invention to provide a dual-purpose implantable system for Vagus-Phrenic Nerve Stimulation (VNS-PhrNS) based on a single endovascular lead and implantable pulse generator (IPG), particularly targeting CHF patients with CSA syndrome, and suitable for integration into a Home Monitoring/Remote Programming therapy regime.

According to a first aspect, this object is achieved by an IPG that is connected or can be connected to a stimulation lead having at least one stimulation electrode. This IPG comprises a respiration activity sensing unit that is configured to generate a signal that represents a respiration activity and at least one stimulation unit that is configured to generate electric stimulation pulses for nerve stimulation. The IPG further comprises a control unit that is configured to trigger delivering of electric stimulation pulses via said at least one stimulation electrode, wherein triggering of the stimulation pulses depends on the signal that represents a respiration activity.

Preferably, the control unit is configured to generate a predictive respiration pattern from the signal that represents a respiration activity and to trigger the stimulation pulses depending on the predictive respiration pattern.

In one embodiment, stimulation pulses can be delivered in anticipation of a physiological event in the respiration pattern that can be predicted from the chest Respiration Effort Signal (REFFS). In a preferred embodiment, the delivery of stimulation occurs during a breathing pause period.

In one embodiment, the control unit is configured to trigger the stimulation pulses using the predictive respiration pattern as feed-forward parameter.

Preferably, the stimulation lead has a plurality of stimulation electrode for delivery of stimulation pulses. The control unit is configured to select one or more electrodes of the plurality of stimulation electrodes and to trigger delivering of electric stimulation pulses via the selected electrodes of the plurality of stimulation electrodes.

In a preferred embodiment, the control unit is configured to trigger delivering of electric stimulation pulses via selected electrodes of the plurality of stimulation electrodes when the signal that represents a respiration activity indicates a breathing pause.

In one embodiment, the signal that represents a respiration activity is a chest Respiration Effort Signal (REFFS).

The invention includes the recognition that an implantable chronic therapy for combined CHF-CSA treatment via VNS-PhrNS should preferably utilize a single endovascular lead, that is implanted through typical pacemaker-lead access sites (e.g. subclavian vein), and that avoids routing through the superior vena cava (SVC) to allow for the possibility of implanting a Cardio Rhythm Management (CRM) device should the patient require one in the future. This makes the azygos vein less of a candidate, in particular because implantable cardioverter defibrillator (ICD) leads, for example, are also implanted in such vein in patients who present high-threshold to stimulation for defibrillation [Bar-Cohen et al. "Novel use of a vascular plug to anchor an azygous vein ICD lead", Journal of Cardiovascular Electrophysiology, 21(1), 99-102, 2010]. The left superior intercostal vein is then the preferred location for the placement of this VNS-PhrNS combo lead. Given the proximity of the vagus branches and phrenic nerve in this location, however, a suitable therapy delivery strategy is required to manage stimulation spillage, which is not addressed in prior art.

In a preferred embodiment, the stimulation lead is a single multi-electrode lead that can be implanted in the left superior intercostal vein and connectable to the IPG that can be located in a pocket in the patient's chest.

The chest Respiration Effort Signal (REFFS) is preferably either derived by an accelerometer in the IPG or by impedance measurement between the IPG case and one of the lead electrodes. Accordingly, it is preferred if the respiration activity sensing unit comprises or is connected to an accelerometer and wherein the respiration activity sensing unit is configured to evaluate an accelerometer signal to thus generate said chest REFFS. Alternatively, the respiration activity sensing unit can comprise or can be connected to an impedance measurement unit that generates an impedance signal that represents intrathoracic impedance and wherein said respiration activity sensing unit is configured to evaluate said impedance signal to thus generate said chest REFFS.

According to a preferred embodiment of the invention, the Respiration Effort Signal (REFFS) is used as feed-forward parameter for the delivery of Vagus Nerve Stimulation (VNS) therapy so any stimulation spillage to the phrenic nerve, should it occur, will assist breathing.

The IPG is preferably connected, or can be connected, to a stimulation lead having a plurality of stimulation electrodes. The control unit of the IPG is preferably configured to trigger delivering of electric stimulation pulses via selected electrodes of the plurality of stimulation electrodes wherein triggering of the stimulation pulses depends on the chest REFFS.

The IPG is preferably configured to perform intrathoracic far-field electrogram (ff-EGM) recordings for CHF monitoring and to detect hypopnea and apnea conditions based on changes to the chest Respiration Effort Signal (REFFS). Upon detection of one of these conditions, Phrenic Nerve Stimulation (PhrNS) can be delivered to restore normal breathing.

In another preferred embodiment, the IPG comprises a heart monitoring unit that is configured to determine at least a heart rate.

The IPG may further comprise a ff-EGM sensing unit that is connected to an electrically conducting case of the IPG and to at least one stimulation electrode of the stimulation lead and that is configured to sense and to evaluate ff-EGM signals.

In a second preferred embodiment, the apparatus operates in a stimulation mode where the chest Respiration Effort Signal (REFFS) is also used as feed-forward parameter for the delivery of Phrenic Nerve Stimulation (PhrNS) therapy. This mode may minimize diaphragm atrophy should the patient be hospitalized and placed on mechanical ventilation.

In yet another preferred embodiment, the IPG has the possibility of communicating via a MICS-band link to an external Programmer, or bedside Patient Messenger connected to a Home Monitoring/Remote Programming Center. For this purpose, the IPG preferably comprises a telemetry unit.

According to a further aspect, the stimulation lead is a percutaneous, linear-type multi-electrode lead that is configured for placement in a left superior intercostal vein.

The inventors found that the left superior intercostal vein provides an ideal location for the dual stimulation of the left phrenic nerve and the cardiac branches of the left vagus nerve. Implantation of a lead in such vein can be done through the left subclavian vein and such lead can be connected to an IPG in a pocket in the left side of the patient's chest. Several electrodes (e.g. eight, similar to a percutaneous spinal cord lead) are preferred for easy lead placement to achieve selective stimulation.

According to a further aspect of the invention, a method for nerve stimulation is provided. The method comprises:
- monitoring respiration activity,
- determining a predictive respiration pattern based on the monitored respiration activity and
- delivering stimulation pulses depending on the monitored respiration activity and/or the predictive respiration pattern for delivering the stimulation pulses.

In a preferred implementation of the method, the stimulation pulses are delivered to a vagus nerve and/or a phrenic nerve. In another implementation, the stimulation pulses are delivered during a breathing pause.

Preferably, the chest Respiration Effort Signal (REFFS) is derived by either an accelerometer in the IPG or by impedance measurement between an electrode in the lead and the IPG case.

Given the proximity of the nerve branches, the chest Respiration Effort Signal (REFFS) is used as feed-forward parameter for the delivery of Vagus Nerve Stimulation (VNS) therapy so any stimulation spillage to the phrenic nerve, should it occur, is preferably timed to assist breathing. In a preferred embodiment, VNS is delivered during the breathing pause period derived from the chest REFFS.

In another preferred embodiment, the IPG is configured so it can record the far-field electrogram (ff-EGM) utilizing a vector between an electrode in the lead and the IPG case. The ff-EGM can be utilized for heart rate determination and heart condition monitoring. Preferably, the IPG comprises a ff-EGM sensing unit that is connected to an electrically-conducting area of the IPG case and to at least one stimulation electrode of the stimulation lead. The ff-EGM sensing unit is configured to sense and to evaluate far-field electrogram signals.

Given apneas can occur at any time of day if the patient falls asleep, monitor and detection of such events are preferably performed independent of the time of day. Preferably, the control unit is configured to deliver Phrenic Nerve Stimulation (PhrNS) therapy upon detection of a hypopnea or an apnea condition from the chest Respiration Effort Signal (REFFS), i.e. shallow breathing effort or absence of breathing effort respectively, to reestablish normal breathing. Although the arrangement of electrodes and stimulation technique for PhrNS will minimize spillage to the vagus nerve (e.g. guarded cathode configuration), spillage is actual beneficial for CHF as both are in the same frequency range.

Preferably, the IPG comprises a triaxial accelerometer. The triaxial capabilities of the accelerometer permits monitoring sleeping positions at night time. This statistic is of particular interest as patients with CHF generally tend to sleep in a sitting position due to breathing difficulties (lungs fill up with fluid). Trend data on sleeping positions, for example, can be utilized to analyze improvements or worsening of a patient's condition. This trend, together with CSA event statistics and detected heart conditions derived from the far-field electrogram (ff-EGM) (e.g. arrhythmias), can be telemetered via a MICS-link to a bedside Patient Messenger which is connected to a Home Monitoring/Remote Programming Center. The same wireless link can be used for programming of the IPG via an external Programmer.

The above and other aspects, features and advantages of the present invention will be more apparent from the following more particular description thereof, presented in conjunction with the following drawings wherein:
- Figure 1: shows an implantable stimulation system according to a preferred embodiment of the invention;
- Figure 2: shows the system of Figure 1 in combination with external devices;
- Figure 3: is a schematic block diagram of some components of an implantable pulse generator (IPG) for use in a system of Figures 1 or 2;
- Figure 4: shows a more detailed sketch of the implanted multi-electrode lead of Figure 1 in the left superior intercostal vein;
- Figure 5: illustrates an alternative embodiment for the lead wherein the four distal electrodes are implemented with opposite active areas;
- Figure 6: illustrates a preferred stimulation arrangement for the electrodes;
- Figure 7: illustrates that the chest Respiration Effort Signal (REFFS) in a CHF patient with CSA alternates two breathing patterns; and
- Figure 8: shows that the mean power at the IPG implant sites (i.e. somewhere between 9-a and 10-a) is 20 dB below the maximum measured at the 11-a position (ideal chest position).

The following description is of the best mode presently contemplated for carrying out the invention. This description is not to be taken in a limiting sense, but is made merely for the purpose of describing the general principles of the invention. The scope of the invention should be determined with reference to the claims.

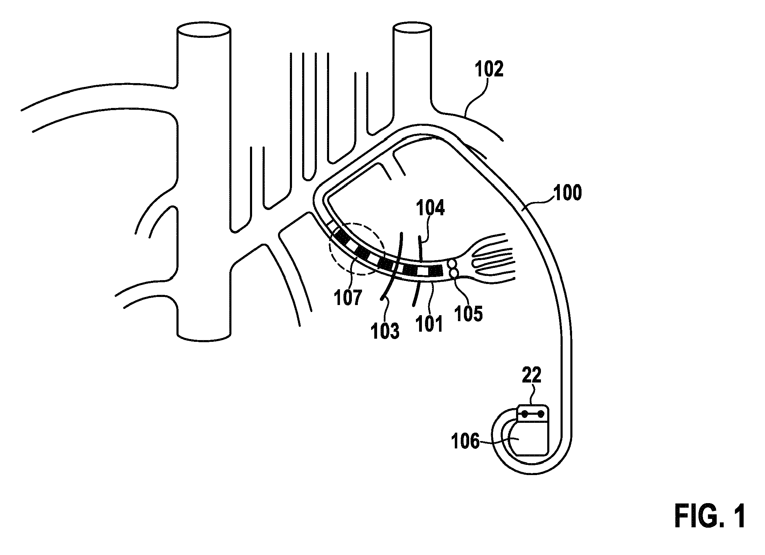

Figure 1 shows an implantable stimulation system comprising a stimulation lead 100 and an implantable pulse generator (IPG) 106 with a header 22.

The stimulation lead 100 is a percutaneous, linear-type multi-electrode lead, which is implanted in the left superior intercostal vein 101 via the subclavian vein 102, following standard pacemaker implantation techniques. Electrodes 107 of the lead 100 form active contact for the delivery of stimulation pulses and/or for impedance measurement. The stimulation lead 100 is positioned in the vein 101 so electrodes are distributed between the phrenic nerve 103 and the vagus nerve 104 which cross such vein 101. The lead 100 has a distal anchoring mechanism 105 and its proximal end is routed subcutaneously and connected to an IPG 106 implanted in the patient's chest.

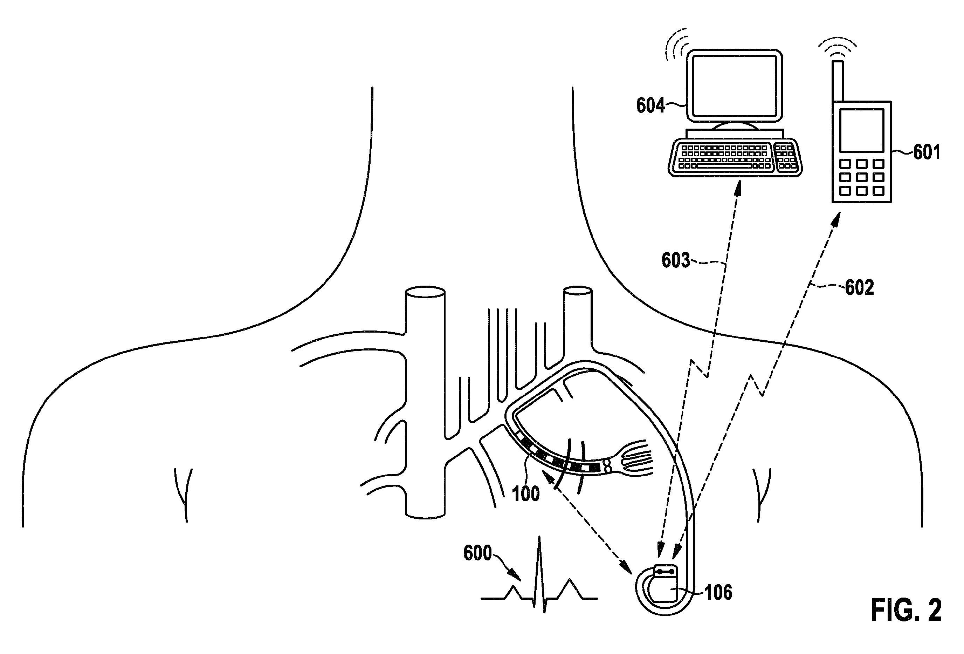

Figure 2 illustrates a preferred system that further includes a bedside Patient Messenger 601 and a Programmer 604 as external devices. The IPG 106 can wirelessly communicate to a bedside Patient Messenger 601 via a MICS-band link 602, which can further relay the information to a Home Monitoring/Remote Programming Center. A similar link 603 allows a Programmer 604 to program the IPG 106.

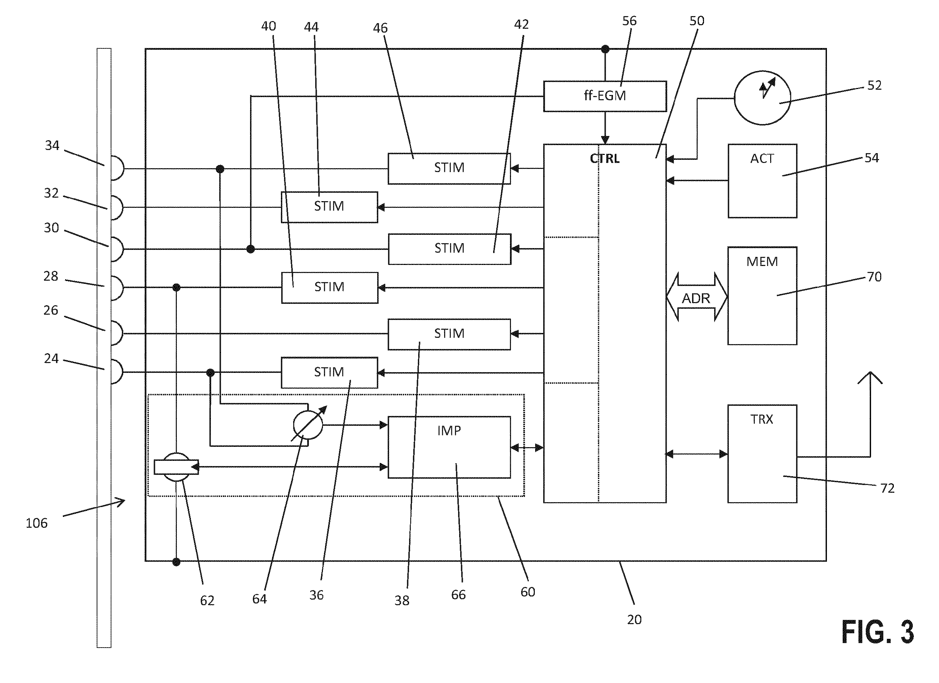

Figure 3 is a schematic diagram of some components of IPG 106.

As can be taken from Figure 3, the IPG 106 comprises a case (IPG case) 20 and a header 22 (see figure 1) for connection of lead 100. Header 22 comprises a number of connectors 24, 26, 28, 30, 32 and 34 that can electrically connect to connectors of stimulation lead 100. Thus, an electric connection between connectors 24, 26, 28, 30, 32 and 34 and electrodes 107 of stimulation lead 100 can be made.

Within IPG case 20 , one or more stimulation units 36, 38, 40, 42, 44 and 46 are arranged electrically connected to connectors 24, 26, 28, 30, 32 and 34 respectively, and configured to generate stimulation pulses and to deliver such stimulation pulses via a respective connector 24, 26, 28, 30, 32 and 34. It should be noted, that instead of one stimulation unit 36 to 46 for each connector 24 to 34 and thus for each electrode 107 of lead 100, one stimulation unit and a switch matrix can be provided. In the latter embodiment, delivery of stimulation pulses via selective connectors and thus via selected electrodes 107 of lead 100 can be achieved by the switch matrix. In another embodiment, all electrodes 107 of lead 100 are switched in parallel to each other and thus only one connector and one stimulation unit is needed. In the latter embodiment, no selection of electrodes 107 for delivery of stimulation pulses is possible.

In the embodiment of Figure 3, each stimulation unit 36, 38, 40, 42, 44 and 46 is connected to and controlled by a control unit 50. Control unit 50 controls generation and triggers delivery of stimulation pulses by stimulation units 36, 38, 40, 42, 44 and 46.

The stimulation pulses to be generated and triggered by each stimulation unit 36, 38, 40, 42, 44 and 46 are tailored for vagus and phrenic nerve stimulation.

Control unit 50 is further connected to a time signal generator 52 that supplies a time base to control unit 50.

Further, an activity sensing unit 54 is provided that is a three-axial accelerometer for sensing movements of the IPG 106 in three spatial dimensions and deliver an accelerometer signal to control unit 50.

Control unit 50 further is connected to a far-field electrogram (ff-EGM) sensing unit 56 that is configured to generate a ff-EGM signal representing a far-field electrogram 600. In order to record such a signal, the ff-EGM sensing unit 56 is connected to at least one of connectors 24 to 34 and thus to one of the electrodes 107 of lead 100. Another input of a ff-EGM sensing unit 56 is connected to the IPG case 20. Thus, a ff-EGM sensing unit 56 can sense voltages between an electrode 107 and the IPG case 20 that result from electric potentials which are caused by the activity of a patient's heart. Far-field electrogram (ff-EGM) sensing unit 56 is configured to supply a ff-EGM signal to control unit 50. The ff-EGM signal represents the heart activity of a patient. From the ff-EGM signal inter alia a heart rate of a patient can be determined.

Control unit 50 is further connected to an impedance measuring unit 60 that comprises a programmable current source 62 for generating and delivering biphasic impedance measuring pulses. Current source 62 may be electrically connected to the IPG case 20 and to at least one of connectors 24 to 34 and thus to at least one of the electrodes 107 of lead 100. Impedance measurement unit 60 further comprises a voltage sensing unit 64 that is configured to measure a voltage difference between an electrode 107 of lead 100 and the IPG case 20, or between two electrodes 107, in response to delivery of current pulses by the current source 62. Current source 62 and voltage sensing unit 64 are connected to an impedance determination unit 66 of the impedance measurement unit 60. Impedance determination unit 66 is configured to generate an impedance signal depending on the voltages measured by voltage sensing unit 64 and to supply such impedance signal to control unit 50. The impedance signal generated by impedance measurement unit 60 represents an intrathoracic impedance which depends on the breathing activity of a patient. Thus, control unit 50 can determine the chest Respiration Effort Signal (REFFS) from the impedance signal supplied by impedance measurement unit 60. Alternatively, as disclosed hereinafter, the chest REFFS can be determined from the accelerometer's signal supplied by activity sensing unit 54.

To extract the chest REFFS from the impedance signal supplied by impedance measurement unit 60, control unit 50 may apply morphological operators as disclosed in US 8,419,645 B2. US 8,419,645 B2 describes how morphological operators can be utilized to determine respiration parameters from a transthoracic impedance signal. Although the measuring configuration disclosed in US 8,419,645 B2 is different from the disclosure in the instant disclosure and thus does not utilize transthoracic impedance given the relative position of the lead 100 with respect to the IPG 106, the method disclosed in US 8,419,645 B2 (column 9, line 5 to column 10, line 26; Figures 2 - 5) can be applied in an analogous manner.

Control unit 50 may be further connected to a memory unit 70 that may serve to store signals recorded by control unit 50 or programs that control the operation of control unit 50.

In order to wirelessly communicate recorded signals to an external device or to receive program instructions, a telemetry unit 72 is provided that is also connected to control unit 50.

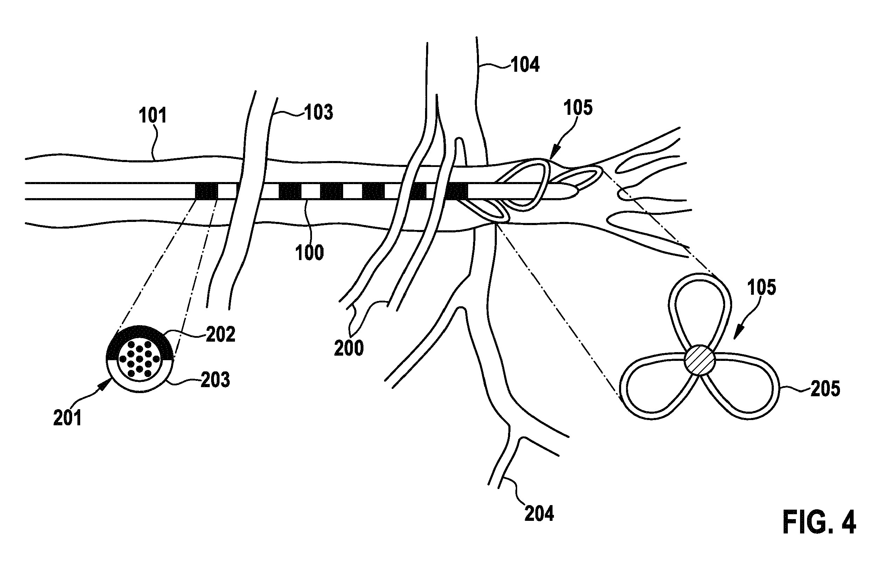

Figure 4 shows a more detailed sketch of the implanted multi-electrode lead 100 in the left superior intercostal vein 101, and of the crossing of the phrenic nerve 103 and the cardiac branches 200 of the vagus nerve 104. Since the target is to stimulate these branches 200 and the phrenic nerve 103, and both typically cross anteriorly to the vein 101, a preferred embodiment for the lead 100 is one where each contact area 201 presents an active area 202 for stimulation and an opposite insulated area 203 (e.g. a ring contact half parylenecoated). The lead 100 can be rotated and implanted so the active areas 202 face the phrenic nerve 103 and the cardiac branches 200. This minimizes unwanted stimulation of the laryngeal nerve branches 204 which forms part of the vagus nerve 104 near the vein 101 crossing. The lead's 100 anchoring mechanism 105 may be based on elastic loops 205 similar to those employed in embolic protection devices.

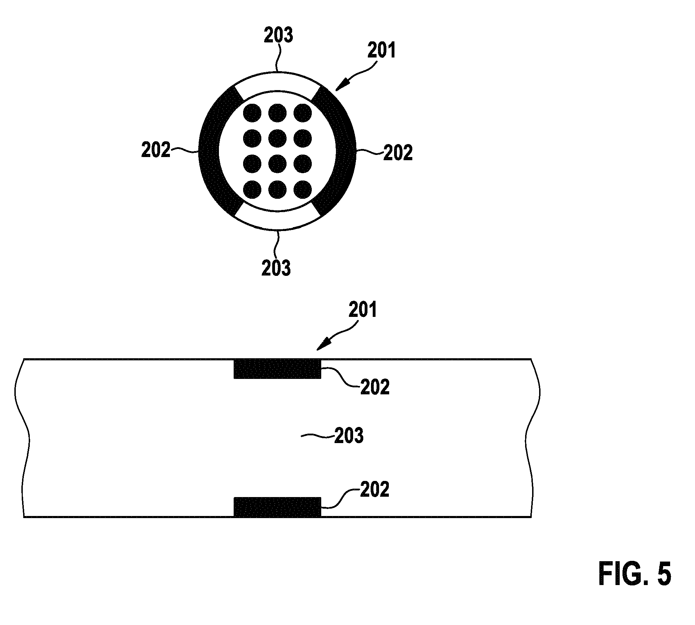

Figure 5 illustrates an alternative embodiment for the lead 100 wherein the contact area 201 of four distal electrodes 107 are implemented with opposite active areas 202, separated by insulating areas 203. In yet another alternative embodiment for the lead 100, the four distal electrodes 107 include three active areas 202 instead separated by insulating areas 203. These alternative lead 100 designs allow for improved stimulation selectivity of the cardiac branches 200 due to anatomical variations that may occur in the branching from the main vagus nerve trunk 104.

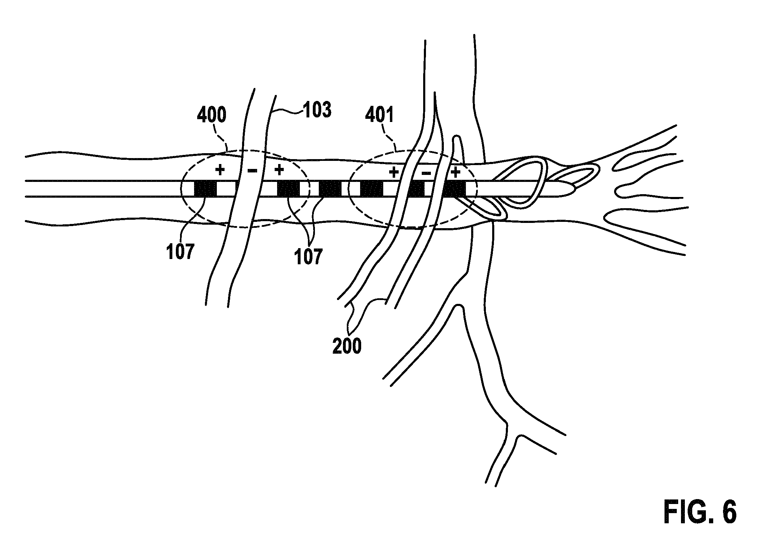

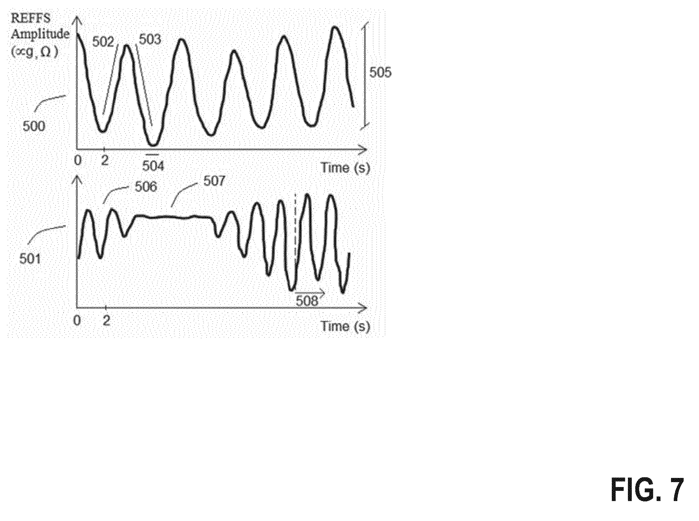

Figure 6 illustrates a preferred stimulation arrangement for the electrodes 107. A first group 400 of electrodes 107 implements a guarded-cathode configuration for the stimulation of the phrenic nerve 103 whereas a second group 401 of electrodes 107 does the same for the stimulation of the vagus nerve cardiac branches 200. The stimulation is preferably current-based and not delivered simultaneously, i.e. Vagus Nerve Stimulation (VNS) is delivered during normal breathing (see next paragraph) as programmed and interrupted when hypopnea 506 or apnea events 507 (see Figure 7) are detected from the chest Respiration Effort Signal (REFFS).

Figure 7 illustrates that the chest REFFS in a CHF patient with CSA alternates two breathing patterns. The top pattern 500 represents normal breathing whereas the bottom 501, known as the Cheyne-Stokes respiration pattern, is typically observed at sleep.

The chest REFFS Amplitude can be proportional to gravity acceleration (ocg), if an accelerometer in the IPG 106 is used, or proportional to ohms (Ω) if impedance measurement between the IPG's case 20 and one electrode 107 in the lead 100 is utilized instead. Normal breathing alternates periods of inspiration 502 and expiration 503, with a breathe pause in between 504, and with a minimum REFFS peak-to-peak amplitude 505 indicative of the patient's normal tidal volume.

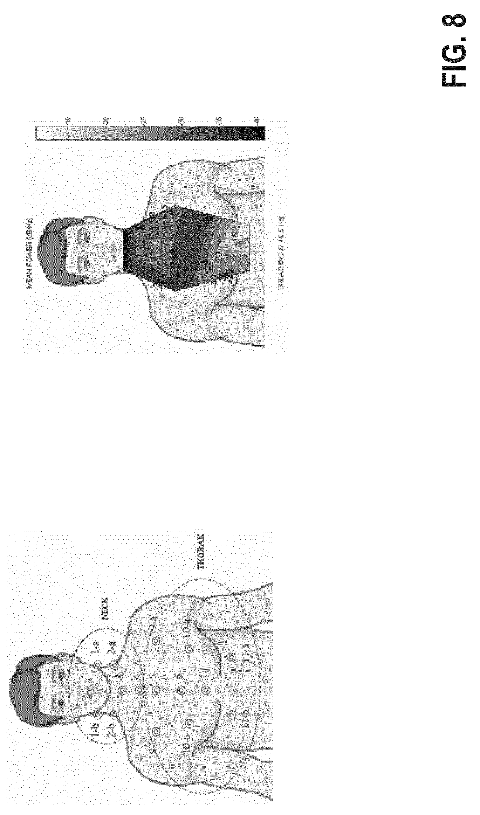

The chest REFFS may be obtained by first band-pass filtering the accelerometer or impedance signal between 0.1 Hz and 0.5 Hz. Figure 8 (adapted from Rendón et al. "Mapping the Human Body for Vibrations using an Accelerometer", Proceedings of the 29th Annual IEEE EMBS International Conference, FrA06.1, pp. 1671-4, 2007) shows that the mean power at the IPG 106 implant sites (i.e. somewhere around 9-a and 10-a) is 20 dB below the maximum measured at the 11-a position (ideal chest position). Hence, signal processing for extraction of the chest REFFS may preferably involve a morphological filter algorithm given the reduced signal-to-noise ratio. In an alternative embodiment, signal processing for extraction of the chest REFFS is based on discrete wavelet transforms. In such scenario, combining signals from both the accelerometer signal supplied by activity sensing unit 54 and the far-field electrogram (ff-EGM) 600 (see last paragraph for a description) may increase the events classification accuracy compared to using the accelerometer signal only.

Vagus Nerve Stimulation (VNS) therapy preferably consists of a programmable train of pulses (including a single pulse) with fixed charge per pulse and frequency between them delivered in anticipation of a physiological event in the respiration pattern that can be predicted from the chest Respiration Effort Signal (REFFS). In a preferred embodiment, the beginning of the delivery of the VNS train of pulses occurs during the breathing pause period 504. Given the delay between Phrenic Nerve Stimulation (PhrNS) and diaphragm muscle contraction, this feed-forward control permits any VNS stimulation spillage to the phrenic nerve (should it occur) to appear at the inspiration period 502 translating into breathing assistance for the patient. VNS may be duty cycled and delivered intermittently, i.e. once every "n" breathing pause periods 504, and only active during programmable daily session times.

When the chest REFFS excursion 505 drops, a hypopnea event 506 may be declared by a detection algorithm in the IPG 106 and PhrNS therapy started during the next breathing pause period 504. To detect entering apnea events 507 directly, a programmable timer 508 from the onset of each inspiration period 502 (normal breathing 500 established) allows starting PhrNS therapy if the following onset of inspiration period 502 cannot be derived from the chest REFFS before the timer 508 elapses.

Vagus Nerve Stimulation (VNS) and Phrenic Nerve Stimulation (PhrNS) are preferably delivered multiplexed in time and the latter has priority over VNS, i.e. if VNS is being delivered and either a hypopnea 506 or an apnea 507 condition is detected from the chest REFFS then VNS may be aborted until PhrNS restores normal breathing 500.

Phrenic Nerve Stimulation (PhrNS) therapy preferably consists of a programmable train of pulses of similar frequency compared to VNS (tens of Hz). Each train may however include a ramp up phase, both in charge injected per pulse and in frequency between pulses, and ramp down phase. This type of train allows for a more natural recruitment of the diaphragm. Once PhrNS allows restoring the chest REFFS to the peak-to-peak amplitude 505, the stimulation is stopped and the chest REFFS monitoring continued.

In another preferred embodiment, the IPG 106 operates in a stimulation mode where the chest Respiration Effort Signal (REFFS) is also used as feed-forward parameter for the delivery of Phrenic Nerve Stimulation (PhrNS) therapy. This may be a magnet-mode triggered by the patient/clinician useful should the patient be hospitalized and placed on mechanical ventilation. Phrenic Nerve Stimulation (PhrNS) may minimize diaphragm atrophy and therefore accelerate the patient weaning off period in such situation. This mode does not prevent Vagus Nerve Stimulation (VNS) therapy from being delivered as it can be time multiplexed with PhrNS.

In yet another preferred embodiment, the triaxial accelerometer may also be used to extract sleeping angle patterns. For example, a decrease in the patient sleeping angle may indicate an improvement of the patient's condition as patients with CHF tend to sleep with several pillows due to breathing difficulties (lungs fill up with fluid) associated with the disease.

Going back to Figure 2, far-field electrogram (ff-EGM) 600 is recorded between an electrode 107 in the lead 100 and the IPG's case 20. Diagnostics and statistics such as sleeping angles, apnea events, arrhythmias, etc. can be wirelessly communicated to a bedside Patient Messenger 601 via a MICS-band link 602, which can further relay the information to a Home Monitoring/Remote Programming Center. A similar link 603 allows a Programmer 604 to program the IPG 106.

A few of the advantages achieved by the system and method of the present invention are:
1) only a single endovascular lead for dual Vagus-Phrenic Nerve Stimulation is needed with reduced side effects;
2) the system is implantable via standard pacemaker techniques and tools hence with reduced risk and minimum Physician training required;
3) the system avoids leads through the SVC/heart chambers and is hence compatible with other CRM devices;
4) the system can provide assistive breathing therapy for CHF patients admitted to the hospital and placed on mechanical ventilation;
5) the system offers added diagnostics/statistics for CHF patients suffering from CSA;
6) ideal therapy for CHF patients suffering from CSA.

Although the present invention has been shown and described in conjunction with several embodiments thereof with reference to the accompanying drawings, it should be apparent to those of ordinary skill that a number of changes and modifications to the invention may be made without departing from the spirit and scope of the invention. In particular, it is possible for IPG 106 to utilize alternative methods of stimulation, e.g. voltage-based stimulation. It is also possible for the lead 100 to include additional electrodes 107 that result located in the access vein when the lead 100 is implanted as described in a preferred embodiment of the present invention. Such additional electrodes may also permit stimulating the vagus and phrenic nerves transvascularly through the access vein wall. This additional feature may further improve stimulation selectivity. This invention can readily be adapted to a number of different kinds of nerve stimulation devices and nerve stimulation methods by following the present teachings. All such changes, modifications and alterations should therefore be recognized as falling within the scope of the present invention.
- 20: IPG case
- 22: header
- 24, 26, 28, 30, 32 and 34: connectors
- 36, 38, 40, 42, 44, and 46: stimulation units
- 50: control unit
- 52: time signal generator
- 54: activity sensing unit
- 56: ff-EGM sensing unit
- 60: impedance measuring unit
- 62: current source
- 64: voltage sensing unit
- 66: impedance determination unit
- 70: memory
- 72: telemetry unit
- 100: multi-electrode lead
- 101: left superior intercostal vein
- 102: subclavian vein
- 103: phrenic nerve
- 104: vagus nerve
- 105: anchoring mechanism
- 106: IPG
- 107: stimulation electrode
- 200: branches
- 201: contact areas
- 202: active area
- 203: insulated area
- 204: branches
- 205: elastic loops
- 400: first group of electrodes 107
- 401: second group of electrodes 107
- 500: normal breathing
- 501: CSA patient respiration pattern
- 502: inspiration period
- 503: expiration period
- 504: breathing pause period
- 505: REFFS amplitude
- 506: hypopnea event
- 507: apnea event
- 508: timer
- 600: ff-EGM
- 601: Patient Messenger
- 602: MICS-band link
- 603: similar link
- 604: Programmer

## Claims

1. Implantable pulse generator (106) that is connected or can be connected to a stimulation lead (100) having at least one stimulation electrode (107),
said implantable pulse generator (106) comprising a respiration activity sensing unit (54; 60) that is configured to generate a signal that represents a respiration activity, said implantable pulse generator (106) further comprising at least one stimulation unit (36, 38, 40, 42, 44, 46) that is configured to generate electric stimulation pulses for nerve stimulation,
said implantable pulse generator (106) further comprising a control unit (50) that is configured to trigger delivering of electric stimulation pulses via said at least one stimulation electrode (107) wherein triggering of the stimulation pulses depends on the signal that represents a respiration activity.

2. Implantable pulse generator (106) according to claim 1, wherein the control unit (50) is configured to generate a predictive respiration pattern from the signal that represents a respiration activity and to trigger the stimulation pulses depending on the predictive respiration pattern.

3. Implantable pulse generator (106) according to claim 1 or 2, wherein the stimulation lead (100) has a plurality of stimulation electrodes (107) for delivery of stimulation pulses and wherein the control unit (50) is configured to select one or more electrodes of the plurality of stimulation electrode (107) and to trigger delivering of electric stimulation pulses via the selected electrode of the plurality of stimulation electrodes (107).

4. Implantable pulse generator (106) according to one of the preceding claims, wherein said control unit (50) is configured to trigger delivering of electric stimulation pulses via selected electrode of the plurality of stimulation electrodes (107) when the signal that represents a respiration activity indicates a breathing pause.

5. Implantable pulse generator (106) according to at least one of the preceding claims, wherein the signal that represents a respiration activity is a chest Respiration Effort Signal (REFFS).

6. Implantable pulse generator (106) according to at least one of the preceding claims, wherein said respiration activity sensing unit (54) comprises or is connected to an accelerometer and wherein said respiration activity sensing unit (54) is configured to evaluate an accelerometer signal to thus generate said chest Respiration Effort Signal (REFFS).

7. Implantable pulse generator (106) according to at least one of the claims 1 to 5, wherein said respiration activity sensing unit (60) comprises or is connected to an impedance measurement unit (60) that generates an impedance signal that represents intrathoracic impedance and wherein said respiration activity sensing unit (60) is configured to evaluate said impedance signal to thus generate said chest Respiration Effort Signal (REFFS).

8. Implantable pulse generator (106) according to at least one of the preceding claims, said implantable pulse generator (106) further comprising a telemetry unit (72) for receiving program instructions and/or for transmitting recorded data and/or status information.

9. Implantable pulse generator (106) according to at least one of the preceding claims, said implantable pulse generator (106) further comprising a heart monitoring unit that is configured to determine at least a heart rate.

10. Implantable pulse generator (106) according to at least one of the preceding claims, said implantable pulse generator (106) further comprising a far-field electrogram (ff-EGM) (600) sensing unit (56) that is connected to an electrically conducting case (20) of the implantable pulse generator (106) and to at least one stimulation electrode (107) of the stimulation lead (100) and that is configured to sense and to evaluate ff-EGM (600) signals.

11. Implantable pulse generator (106) according to at least one of the preceding claims, wherein said stimulation lead (100) is a percutaneous, linear-type, multi-electrode lead that is configured for placement in a left superior intercostal vein.

12. Method for nerve stimulation, said method comprising:
- monitoring respiration activity,
- determining a predictive respiration pattern based on the monitored respiration activity and
- delivering stimulation pulses depending on the monitored respiration activity and/or the predictive respiration pattern for delivering the stimulation pulses.

13. Method for nerve stimulation according to claim 12, wherein the stimulation pulses are delivered to a vagus nerve and/or a phrenic nerve.

14. Method for nerve stimulation according to claim 12 or 13, wherein the stimulation pulses are delivered during a breathing pause (504).
